# EUROPÄISCHE PATENTANMELDUNG

(11) **EP 4 226 894 A1**
(43) Veröffentlichungstag der Anmeldung: **16.08.2023**
(21) Anmeldenummer: 23172210.9
(22) Anmeldetag: 16.12.2020
(51) Int. Cl.: A61C 13/225, A61C 13/24

(54) **DENTALOBJEKT MIT EINEM HAFTBEREICH**

(62) Teilanmeldung aus: 20214629.6
(71) Anmelder: Ivoclar Vivadent AG, 9494 Schaan (LI)
(72) Erfinder: REINHARDT, Jonas, 7206 Igis (CH); RAMPF, Markus, 7212 Seewis-Dorf (CH); GROSSE-HONEBRINK, Alexander, 9320 Arbon (CH); TICHY, Raimund, 6800 Feldkirch (AT)
(74) Vertreter: Baldus, Oliver

(57) **Zusammenfassung**

Die vorliegende Erfindung betrifft ein Dentalobjekt (100), mit einem Haftbereich (103) zum Befestigen des Dentalobjekts (100) an einem Zahn, Zähnen (105) oder dem Zahnfleisch, der eine Anordnung (101) von Saugnäpfen (107-1, ..., 107-n) umfasst und der Haftbereich (103), das Dentalobjekt (100) und/oder ein Gehäuse (115) ein oder mehrere Kanäle (125) zum Führen von Speichel zu einer Sensoreinheit umfasst.

## Beschreibung

Die vorliegende Erfindung betrifft ein Dentalobjekt mit einem Haftbereich und ein Verfahren zum Befestigen eines Dentalobjekts.

Heutige intraorale Befestigungsmöglichkeiten sind mechanisch durch Klemmen oder chemisch durch ein Verkleben (Adhäsion) realisiert. Bekannte Klemmverfahren beinhalten beispielsweise Klemmen, die um einen oder mehrere Zähne gelegt werden, dentale Aligner, die den gesamten oder Teile des Zahnbogens umfassen, oder molare Bänder, die um einen oder mehrere Zähne gelegt werden. Eine Adhäsion wird mittels Klebemittel oder Zement erreicht. Eine Befestigung mittels Adhäsion ist nicht einfach entfernbar, sondern muss von einem Zahnarzt entfernt werden und ist nicht ohne Schaden am Zahnschmelz möglich. Dies kann zu Problemen für die Patienten führen, da sie einen Fremdkörper zur Mundhygiene nicht selbst entfernen können und dies nicht ohne Schaden an gesundem Zahnmaterial möglich ist.

Eine weitere Befestigungsmöglichkeit kann mit einer Haftcreme erreicht werden. Eine Haftcreme funktioniert aber nur auf dem Zahnfleisch, jedoch nicht auf dem Zahn selbst. Die Haftcreme kann nur für große Flächen (Full Denture) verwendet werden. Weiter verursacht Haftcreme nur schwache Befestigung, so dass das Tragen eines verschluckbaren Fremdkörpers über die Nacht nicht möglich ist. Klemmverfahren können demgegenüber die Zahnsubstanz beschädigen oder durch Druck sogar Zähne verschieben.

Es ist die technische Aufgabe der vorliegenden Erfindung, eine stark haftende und reversible Befestigung eines Dentalobjekts im Intraoralraum zu ermöglichen.

Diese Aufgabe wird durch Gegenstände nach den unabhängigen Ansprüchen gelöst. Technisch vorteilhafte Ausführungsformen sind Gegenstand der abhängigen Ansprüche, der Beschreibung und der Zeichnungen.

Gemäß einem ersten Aspekt wird die technische Aufgabe durch ein Dentalobjekt gelöst, mit einem Haftbereich zum Befestigen des Dentalobjekts an einem Zahn, Zähnen oder einem Zahnfleisch, der eine Anordnung von Saugnäpfen umfasst und der Haftbereich, das Dentalobjekt und/oder ein Gehäuse ein oder mehrere Kanäle zum Führen von Speichel zu einer Sensoreinheit umfasst. Der technische Vorteil von vielen kleinen Saugnäpfen ist, dass diese auch auf gekrümmten Flächen eine hohe Haftung ermöglichen, da eine Zahnkrümmung im Mikrometerbereich flach erscheint. Es ist hierzu ausreichend, wenn nur ein Teil der Saugnäpfe am Zahn haftet, um eine genügend gute Gesamthaftung zu gewährleisten. Zudem ist durch das Dentalobjekt eine Entfernbarkeit der Befestigung gegeben, damit ein Patient nach einem Entfernen des Dentalobjektes einer gewohnten Mundhygiene nachgehen kann und keine Sekundärerkrankungen durch das Dentalobjekt ausgelöst werden. Dadurch kann eine Plaquebildung unter dem oder um das Dentalobjekt nach einem Entfernen gereinigt werden. Der Patient ist in der Lage, das Dentalobjekt jederzeit zu entfernen und wiedereinzusetzen. Dabei werden die Zähne nicht geschädigt, da keine Ätzung, keine Klemmen, keine Bügel, kein Klebstoff und kein Metall verwendet werden. Die Zähne werden durch das Dentalobjekt nicht verschoben, wie beispielsweise bei einem molaren Band und es wird keine Haftcreme benötigt. Da das Dentalobjekt nur an der Seite angebracht ist gibt es keine Bisserhöhung was bei Klammern oder Bissschienen der Fall ist.

In einer technisch vorteilhaften Ausführungsform des Dentalobjekts weisen die Saugnäpfe einen äußeren Durchmesser von 10 nm bis 5 mm auf, bevorzugt 10 bis 50 µm. Dadurch wird beispielsweise der technische Vorteil erreicht, dass eine hohe Haftwirkung am Zahn erreicht wird.

In einer weiteren technisch vorteilhaften Ausführungsform des Dentalobjekts sind die Saugnäpfe aus Polyurethan-Acrylat-basiertem Polymer oder Polyethylen-Acrylat-basiertem Polymer hergestellt. Dadurch wird beispielsweise der technische Vorteil erreicht, dass ein Material mit einer besonders großen Haftung verwendet wird.

In einer weiteren technisch vorteilhaften Ausführungsform des Dentalobjekts sind die Saugnäpfe glockenförmig, kugelförmig oder zylinderförmig ausgebildet. Dadurch wird beispielsweise der technische Vorteil erreicht, dass der Rand der Saugnäpfe sich besser an den Zahn anpasst.

In einer weiteren technisch vorteilhaften Ausführungsform des Dentalobjekts weisen die Saugnäpfe einen zentralen Vorsprung auf. Dadurch wird beispielsweise der technische Vorteil erreicht, dass sich die Saugnäpfe leichter wieder ablösen lassen.

In einer weiteren technisch vorteilhaften Ausführungsform des Dentalobjekts ist der zentrale Vorsprung kugelförmig oder konisch. Dadurch wird beispielsweise ebenfalls der technische Vorteil erreicht, dass sich die Saugnäpfe leichter wieder ablösen lassen.

In einer weiteren technisch vorteilhaften Ausführungsform des Dentalobjekts weist das Dentalobjekt eine Kontaktfläche mit einer konkaven Aussparung zum Einfügen eines Zahnes auf, an der der Haftbereich angeordnet ist. Dadurch wird beispielsweise ebenfalls der technische Vorteil erreicht, dass eine anatomische Anpassung des Dentalobjektes erfolgt.

In einer weiteren technisch vorteilhaften Ausführungsform des Dentalobjekts umfasst die Anordnung erste Saugnäpfe mit einem ersten Durchmesser und zweite Saugnäpfe mit einem zweiten Durchmesser. Dadurch wird beispielsweise ebenfalls der technische Vorteil erreicht, dass die Haftwirkung am Zahn verbessert werden kann.

In einer weiteren technisch vorteilhaften Ausführungsform des Dentalobjekts sind die Saugnäpfe durch zylinderförmige Aussparungen in dem Haftbereich gebildet. Dadurch wird beispielsweise ebenfalls der technische Vorteil erreicht, dass die Herstellung der Saugnäpfe auf einfachere Weise hergestellt werden kann.

In einer weiteren technisch vorteilhaften Ausführungsform des Dentalobjekts weist der Haftbereich eine Dicke von 10 µm bis 5 mm auf, bevorzugt 0,5 bis 1,5 mm. Dadurch wird beispielsweise ebenfalls der technische Vorteil erreicht, dass eine besonders gute Anpassung an den Zahn erfolgt.

In einer weiteren technisch vorteilhaften Ausführungsform des Dentalobjekts sind die Saugnäpfe in einer hexagonalen Anordnung angeordnet. Dadurch wird beispielsweise ebenfalls der technische Vorteil erreicht, dass eine gleichmäßige Haftwirkung und gute Flächennutzung erzielt wird.

In einer weiteren technisch vorteilhaften Ausführungsform des Dentalobjekts ist der Haftbereich mittels einer Klebschicht an einer Kontaktfläche befestigt und/oder der Haftbereich umfasst ein Verbindungselement zum Herstellen einer Formschlussgeometrie mit einem Sensor und/oder einem Sensorgehäuse und/oder der Haftbereich umfasst ein Rastelement zum Einrasten an einem Sensor und/oder Sensorgehäuse. Das Verbindungselement kann ein Noppen sein. Dadurch wird beispielsweise ebenfalls der technische Vorteil erreicht, dass die Haftung des Haftbereichs an der Kontaktfläche verbessert wird und Dentalobjekte befestigt werden können.

In einer weiteren technisch vorteilhaften Ausführungsform des Dentalobjekts umfasst der Haftbereich antibakterielle Partikel. Dadurch wird beispielsweise ebenfalls der technische Vorteil erreicht, dass eine Entzündung beim Tragen des Dentalobjekts verhindert wird.

In einer weiteren technisch vorteilhaften Ausführungsform des Dentalobjekts umfasst der Haftbereich, das Dentalobjekt und/oder das Gehäuse ein oder mehrere Kanäle zum Führen von Speichel zu einer Sensoreinheit. Dadurch wird beispielsweise ebenfalls der technische Vorteil erreicht, dass Eigenschaften des Speichels durch das Dentalobjekt analysiert werden können.

In einer weiteren technisch vorteilhaften Ausführungsform des Dentalobjekts umfasst das Dentalobjekt eine elektronische Sensoreinheit und/oder ein Sensorgehäuse. Dadurch wird beispielsweise der technische Vorteil erreicht, dass ein Sensor zum Erfassen von Daten im Intraoralraum angeordnet werden kann.

Gemäß einem ersten Aspekt wird die technische Aufgabe durch ein Verfahren zum Befestigen eines Dentalobjekts, mit dem Schritt eines Aufdrückens eines Haftbereichs des Dentalobjekts an einen Zahn, Zähnen oder dem Zahnfleisch, der eine Anordnung von Saugnäpfen umfasst, wobei der Haftbereich, das Dentalobjekt und/oder ein Gehäuse ein oder mehrere Kanäle zum Führen von Speichel zu einer Sensoreinheit umfasst. Dadurch werden die gleichen technischen Vorteile wie durch das Dentalobjekt nach dem ersten Aspekt erreicht.

Ausführungsbeispiele der Erfindung sind in den Zeichnungen dargestellt und werden im Folgenden näher beschrieben.

Es zeigen:
- Fig. 1: eine schematische Darstellung eines Dentalobjekts und eine Anordnung von Saugnäpfen;
- Fig. 2: eine Darstellung von zwei Saugnäpfen;
- Fig. 3: eine schematische Darstellung des Haftbereiches mit Kanälen für einen Flüssigkeitsdurchfluss;
- Fig. 4: eine schematische Darstellung eines Haftbereiches mit Saugnäpfen unterschiedlichen Durchmessers; und
- Fig. 5A-5D: Querschnitte durch unterschiedliche Gestaltungen von Saugnäpfen.

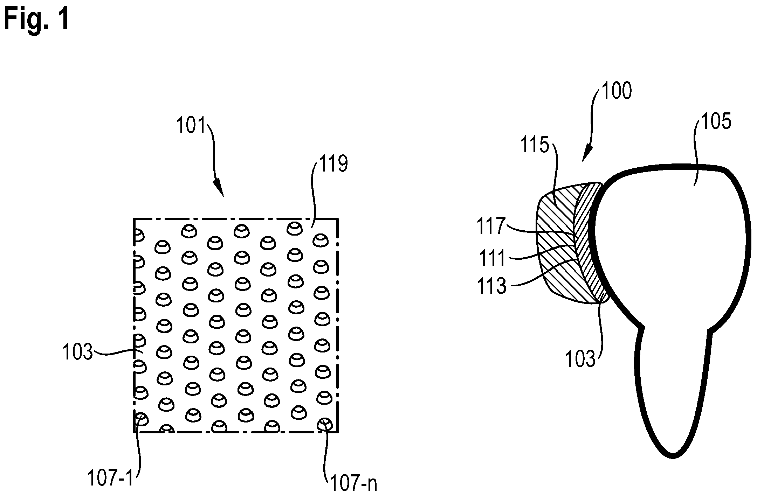

Fig. 1 zeigt eine schematische Darstellung eines Dentalobjekts 100 mit einem Haftbereich 103 zum Befestigen des Dentalobjekts 100 an einem Zahn 105. Der Haftbereich 103 des Dentalobjekts 100 umfasst eine matrixförmige Anordnung 101 von Saugnäpfen 107-1, ..., 107-n. Die Saugnäpfe 107-1, ..., 107-n dienen dazu, um das Dentalobjekt 100 in der Mundhöhle zu fixieren. Der Haftbereich 103 ist an einer Kontaktfläche 111 des Dentalobjekts 100 befestigt.

Die Anzahl der Saugnäpfe 107-1, ..., 107-n pro cm² beträgt beispielsweise zwischen 30.000 bis 40.000 Stück. Der Durchmesser D der Saugnäpfe 107-1, ..., 107-n beträgt beispielsweise 50 µm. Allerdings können auch in unterschiedlichen Bereichen des Haftbereichs 103 verschiedene Saugnäpfe 107-1, ..., 107-n mit unterschiedlichen Durchmessern angeordnet sein. Die Anordnung 101 kann beispielsweise erste Saugnäpfe 107-1, ..., 107-n mit einem ersten Durchmesser und zweite Saugnäpfe mit einem zweiten Durchmesser aufweisen. Dadurch kann die Haftung des Haftbereich 103 auf die verschiedenen Bereiche des Zahns 105 angepasst werden. Die Anordnung 101 der Saugnäpfe 107-1, ..., 107-n liegt beispielsweise in hexagonaler oder matrixartiger Form vor. Die Anordnung 101 der Saugnäpfe 107-1, ..., 107-n kann jedoch auch unregelmäßig sein.

Der Haftbereich 103 kann aus Polyurethan-Acrylat-basiertem Polymer oder Polyethylen-Acrylat-basiertem Polymer hergestellt sein und antibakterielle Partikel umfassen, wie beispielsweise durch einen Einbau von Silberpartikeln, Kupferpartikeln oder eine Mischung mit Chlorhexidin and Chloroxylenol. Zudem kann das plastische Material Antibiotika umfassen, wie beispielsweise Penicillin, Clindamycin, Erythromycin, Cefadroxil, Metronidazol und/oder Tetracycline. Zudem können pH-regulierende Füllmaterialen verwendet werden, wie Kalziumfluorid, Kalziumhydroxid oder alkalische Glasfüller, wie Kalzium-Fluorosilicat-Glas oder ionenfreisetzende Materialien, wie beispielsweise von F-, OH-, oder Ca2+.

Das Dentalobjekt 100 kann folgende Bestandteile umfassen: Sensoren, Elektronikbauteile, Antennen, Batterien, oder Gehäuse für verschiedene Bestandteile. Das Gehäuse 115 kann aus einem Kunststoff, Karbon oder Metall gefertigt sein. Das Dentalobjekt 100 kann ein Objekt sein, das bereits eine anatomisch auf den Zahn 105 angepasste Form aufweist.

Ein derartiges Objekt kann beispielsweise mittels eines 3D-Druckers individuell erzeugt und an die spezielle Form eine Zahns 105 eines Patienten angepasst sein, wie beispielsweise ein individuell geformter Zahnaufsatz. Im Allgemeinen kann das Dentalobjekt 100 jedes Objekt sein, das im Mundraum an dem Zahn 105 befestigt werden soll. Die Saugnäpfe 107-1, ..., 107-n können beispielsweise mittels eines 3D-Druckverfahrens, eines Heißprägeverfahrens oder eines Spritzgussverfahrens hergestellt werden.

Das Dentalobjekt 100 und/oder der Haftbereich 103 können anatomisch vorgeformt sein, indem diese eine konkave Einwölbung 117 umfassen, in der die Zähne 105 liegen. Dadurch liegt das Dentalobjekt 100 besser an den Zähnen 105 an. In dem Haftbereich 103 kann zudem eine vorgefertigte Durchgangsöffnung zum Führen von Flüssigkeit (Speichel) vom Zahn 105 in das Innere des Gehäuses 115 vorgesehen sein. Die Flüssigkeit wird über die Durchgangsöffnung zum Sensor geführt, damit eine definierte Flussmenge an diesem ankommt. Dadurch kann die Flüssigkeit von dem Zahn 105 im Inneren des Gehäuses 115 von einem Sensor analysiert werden. Daneben ist es möglich, ein gezieltes Mikroklima zu erzeugen, das auf den Messwert abgestimmt ist.

Bei individuellen Dentalobjekten 100, die räumlich und anatomisch an den Zahn 105 angepasst sind, kann die Dicke des Haftbereichs 103 gering sein und in einem Bereich von 200 µm liegen. Bei vorgefertigten, standardmäßigen Dentalobjekten 100 liegt die Dicke des Haftbereichs 103 im Bereich von Millimetern, da eine größere plastische Verformung des Haftbereichs 103 stattfindet. Die Abmessungen des Haftbereichs 103 in Länge und Breite können beispielsweise 10 mm mal 10 mm betragen. Im Allgemeinen können jedoch auch andere Abmessungen gewählt werden, sofern diese zweckdienlich sind.

Durch den Haftbereich 103 können nicht-prothetische, nichtorthodontische Dentalobjekte 100 (Appliance) im Intraoralbereich reversibel befestigt werden. Aufgrund der Befestigung mit den Saugnäpfen 107-1, ..., 107-n kann das Dentalobjekt 100 jederzeit entfernt werden und auch wiedereingesetzt werden.

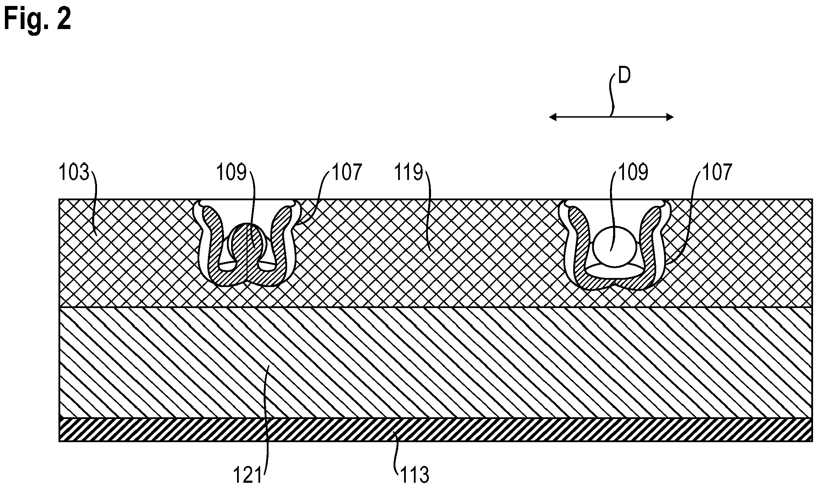

Fig. 2 zeigt eine Darstellung zweier Saugnäpfe 107. Die Saugnäpfe 107-1, ..., 107-n können glockenförmig ausgebildet sein, so dass sich der Rand der Saugnäpfe 107-1, ..., 107-n nach außen hin aufweitet. Die Saugnäpfe 107-1, ..., 107-n sind topfförmig ausgebildet und weisen in der Mitte einen zentralen, kugelförmigen oder konischen Vorsprung 109 auf, der im Inneren der Saugnäpfe 107-1, ..., 107-n liegt. Der Vorsprung 109 sorgt für eine erhöhte Haftkraft bei feuchten Bedingungen, da dieser für die Aufrechterhaltung eines Flüssigkeitsfilms im Inneren der Saugnäpfe 107-1, ..., 107-n sorgt. Der Vorsprung 109 erzeugt somit eine gesteigerte Kohäsionskraft durch die Flüssigkeitsmoleküle.

Die Saugnäpfe 107-1, ..., 107-n können auf verschiedene Weise in dem Haftbereich 103 hergestellt werden. Beispielsweise werden die Saugnäpfe 107-1, ..., 107-n in eine dünne Folie (~ 50 pm) 119 eingebracht, die auf einer Seite am Gehäuse 115 durch eine Klebschicht 113 befestigt ist und auf der anderen Seite die Saugnäpfe 107-1, ..., 107-n aufweist. Diese Folie kann dann individuell auf dem Dentalobjekt 100 verklebt werden, so dass das Dentalobjekt 100 mit der Saugnapffolie auf dem Zahn 105 befestigt werden kann.

An dem Haftbereich 103 können ein oder mehrere positiv oder negativ ausgestaltete Verbindungselemente vorgesehen sein, die eine Formschlussgeometrie mit einem Dentalobjekt 100 herstellen. Auf diese Weise kann an dem Haftbereich 103 ein Dentalobjekt 100 oder Gehäuse 115 befestigt werden. Das Dentalobjekt 100 oder das Gehäuse 115 weisen hierbei positiv oder negativ ausgestaltete Verbindungselemente auf, die zusammen mit dem Verbindungselement des Haftbereiches 103 den Formschluss bewirken. Es können ein oder mehrere Rastelemente vorgesehen sein, an denen Sensor oder das Gehäuse 115 an dem Haftbereich 103 befestigt wird.

Zusätzlich kann der Haftbereich 103 zwischen der Folie 119 und dem Gehäuse 115 eine deformierbare Schicht 121 umfassen, die sich beim Andrücken des Dentalobjekts 100 an den Zahn 105 verformt. Die deformierbare Schicht 121 ist beispielsweise aus einem elastisch verformbaren Schaumstoff oder Gummi oder einem plastisch verformbaren Material gebildet, wie beispielsweise Plastilin. Diese wird mittels der Klebschicht 113 mit dem Gehäuse 115 des Dentalobjektes 100 verbunden.

Bei dem Befestigen der Saugnäpfe 107-1, ..., 107-n ist eine feuchte Umgebung (Speichel) von Vorteil, da die Dichtung durch die Feuchtigkeit erhöht wird. Die Abzugskräftemessungen zeigen, dass die Anordnung 101 von Saugnäpfe 107-1, ..., 107-n besser am Zahn 105 haftet als Haftcreme.

Das Verfahren zum Befestigen des Dentalobjekts 100 umfasst den Schritt des Aufdrückens des Haftbereichs 103 des Dentalobjekts 100 an einen Zahn 105, der eine matrixförmige Anordnung 101 von elastischen Saugnäpfen 107-1, ..., 107-n umfasst. Durch das Aufdrücken saugen sich die Saugnäpfe 107-1, ..., 107-n am Zahn 105 fest und halten das Dentalobjekt 100 an seiner Position.

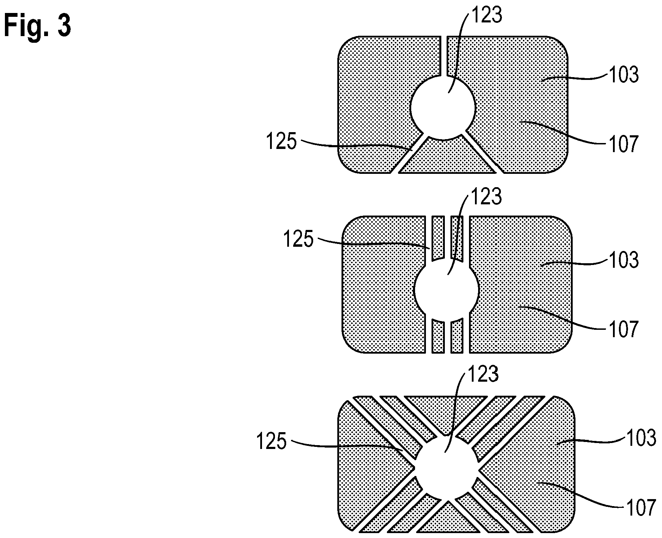

Fig. 3 zeigt eine schematische Darstellung des Haftbereiches 103 mit Kanälen 125 für einen Flüssigkeitsdurchfluss. In dem Haftbereich 103, dem Dentalobjekt 100 und/oder dem Gehäuse 115 ist die Durchgangsöffnung 123 zum Ermöglichen einer Messung durch Sensoreinheit angeordnet. Die Durchgangsöffnung 123 bildet einen Messbereich für die Sensoreinheit.

Die Kanäle 125 sind in dem Haftbereich 103, dem Dentalobjekt 100 und/oder dem Gehäuse 115 durch Vertiefungen gebildet und dienen dazu Flüssigkeit (Speichel) zu der Sensoreinheit hinzuführen und eine Ventilation des Messbereichs zu ermöglichen. Die Kanäle 125 können in horizontaler, diagonaler oder vertikaler Richtung angeordnet sein.

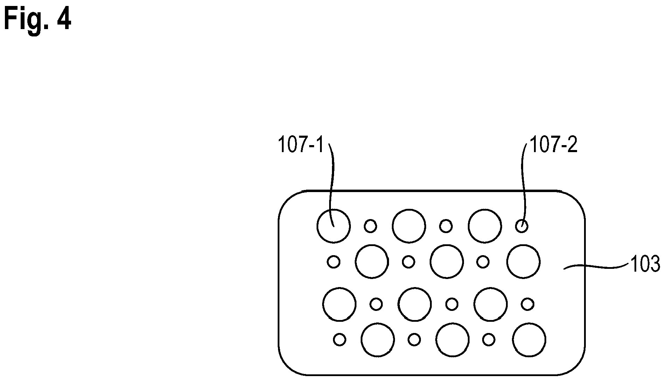

Fig. 4 zeigt eine schematische Darstellung eines Haftbereiches 103 mit Saugnäpfen 107 unterschiedlichen Durchmessers. Der Haftbereich 103 umfasst Saugnäpfe 107-1 und 107-2, die jeweils einen unterschiedlichen äußeren Durchmesser aufweisen. Der Durchmesser der Saugnäpfe 107-1 ist größer als der Durchmesser der Saugnäpfe 107-2. Dadurch kann die Haftwirkung am Zahn verbessert werden, dass der Haftbereich 103 für unterschiedliche Oberflächen angepasst sein kann. Falls auch einer Oberfläche die Saugnäpfe 107-1 nicht ausreichend halten, kann die Haftwirkung über die kleineren Saugnäpfe 107-2 erreicht werden und umgekehrt.

Im Allgemeinen können die Saugnäpfe 107 aber auch noch weitere Durchmesser aufweisen oder sogar in dem Durchmesser sogar zufällig verteilt sein. In diesem Fall weisen die Saugnäpfe 107 zufällige Durchmesser innerhalb eines bestimmten Bereichs auf.

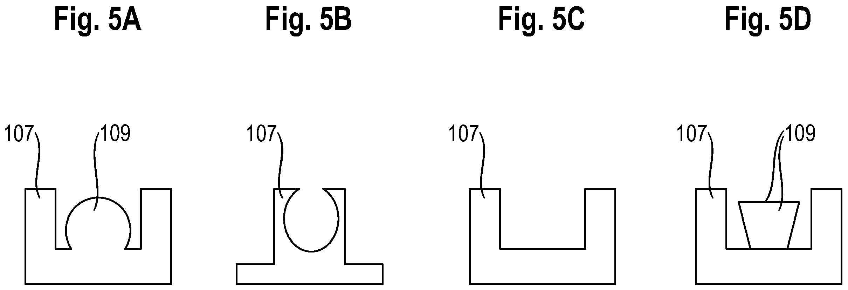

Fig. 5A-5D zeigen Querschnitte durch unterschiedliche Gestaltungen von Saugnäpfen 107. In Fig. 5A ist der Saugnapf 107 zylinderförmig ausgebildet. In der Mitte weist dieser einen kugelförmigen Vorsprung 109 auf, der sich im Inneren der zylinderförmigen Aussparung des Saugnapfes 107 befindet. In Fig. 5B ist der Saugnapf 107 kugelförmig ausgebildet. Der Saugnapf 107 weist eine kugelförmige Aussparung auf. In Fig. 5C ist der Saugnapf 107 ebenfalls zylinderförmig ausgebildet, ohne dass dieser einen Vorsprung 109 aufweist. In Fig. 5D ist der Saugnapf 107 ebenfalls zylinderförmig ausgebildet. In der Mitte weist dieser einen umgekehrt konischen Vorsprung 109 auf, der sich im Inneren der zylinderförmigen Aussparung des Saugnapfes 107 befindet und dessen größere Grundfläche 127 nach außen zeigt.

Die Anordnung (Array) 101 von Saugnäpfen 107-1, ..., 107-n wird intra-oral für die abnehmbare Befestigung des Dentalobjekts 100 verwendet. Durch Die Verwendung von zahlreichen Mikrosaugnäpfen wird eine flexible Befestigung ermöglicht.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Alle Verfahrensschritte können durch Vorrichtungen implementiert werden, die zum Ausführen des jeweiligen Verfahrensschrittes geeignet sind. Alle Funktionen, die von gegenständlichen Merkmalen ausgeführt werden, können ein Verfahrensschritt eines Verfahrens sein.

Der Schutzbereich der vorliegenden Erfindung ist durch die Ansprüche gegeben und wird durch die in der Beschreibung erläuterten oder den Figuren gezeigten Merkmale nicht beschränkt.

### BEZUGSZEICHENLISTE

- 100: Dentalobjekt
- 101: Anordnung
- 103: Haftbereich
- 105: Zahn
- 107: Saugnäpfe
- 109: Vorsprung
- 111: Kontaktfläche
- 113: Klebschicht
- 115: Gehäuse
- 117: Einwölbung
- 119: Folie
- 121: plastische Schicht
- 123: Durchgangsöffnung
- 125: Kanal
- 127: Grundfläche

## Patentansprüche

1. Dentalobjekt (100), mit;
einem Haftbereich (103) zum Befestigen des Dentalobjekts (100) an einem Zahn, Zähnen (105) oder einem Zahnfleisch, der eine Anordnung (101) von Saugnäpfen (107-1, ..., 107-n) umfasst und der Haftbereich (103), das Dentalobjekt (100) und/oder ein Gehäuse (115) ein oder mehrere Kanäle (125) zum Führen von Speichel zu einer Sensoreinheit umfasst.

2. Dentalobjekt (100) nach Anspruch 1, wobei die Saugnäpfe (107-1, ..., 107-n) einen äußeren Durchmesser von 10 nm bis 5 mm aufweisen.

3. Dentalobjekt (100) nach einem der vorangehenden Ansprüche, wobei die Saugnäpfe (107-1, ..., 107-n) aus Polyurethan-Acrylat-basiertem Polymer oder Polyethylen-Acrylat-basiertem Polymer hergestellt sind.

4. Dentalobjekt (100) nach einem der vorangehenden Ansprüche, wobei die Saugnäpfe (107-1, ..., 107-n) glockenförmig, kugelförmig oder zylinderförmig ausgebildet sind.

5. Dentalobjekt (100) nach einem der vorangehenden Ansprüche, wobei die Saugnäpfe (107-1, ..., 107-n) einen zentralen Vorsprung (109) aufweisen.

6. Dentalobjekt (100) nach Anspruch 5, wobei der zentrale Vorsprung (109) kugelförmig oder konisch ist.

7. Dentalobjekt (100) nach einem der vorangehenden Ansprüche, wobei das Dentalobjekt (100) eine Kontaktfläche (111) mit einer konkaven Aussparung zum Einfügen eines Zahnes (105) aufweist, an der der Haftbereich (103) angeordnet ist.

8. Dentalobjekt (100) nach einem der vorangehenden Ansprüche, wobei die Anordnung (101) erste Saugnäpfe (107-1, ..., 107-n) mit einem ersten Durchmesser und zweite Saugnäpfe mit einem zweiten Durchmesser umfasst.

9. Dentalobjekt (100) nach einem der vorangehenden Ansprüche, wobei die Saugnäpfe (107-1, ..., 107-n) durch zylinderförmige Aussparungen in dem Haftbereich (103) gebildet sind.

10. Dentalobjekt (100) nach einem der vorangehenden Ansprüche, wobei der Haftbereich (103) eine Dicke von 10 µm bis 5 mm aufweist.

11. Dentalobjekt (100) nach einem der vorangehenden Ansprüche, wobei die Saugnäpfe (107-1, ..., 107-n) in einer hexagonalen Anordnung (101) angeordnet sind.

12. Dentalobjekt (100) nach einem der vorangehenden Ansprüche, wobei der Haftbereich (103) mittels einer Klebschicht (113) an einer Kontaktfläche (111) befestigt ist und/oder der Haftbereich (103) ein Verbindungselement zum Herstellen einer Formschlussgeometrie mit einem Sensor und/oder einem Sensorgehäuse und/oder der Haftbereich (103) ein Rastelement zum Einrasten an einem Sensor und/oder Sensorgehäuse umfasst.

13. Dentalobjekt (100) nach einem der vorangehenden Ansprüche, wobei das Dentalobjekt (100) eine elektronische Sensoreinheit und/oder ein Sensorgehäuse umfasst.

14. Verfahren zum Befestigen eines Dentalobjekts (100), mit dem Schritt:
Aufdrücken eines Haftbereichs (103) des Dentalobjekts (100) an einen Zahn (105), Zähne oder dem Zahnfleisch, der eine Anordnung (101) von Saugnäpfen (107-1, ..., 107-n) umfasst, wobei der Haftbereich (103), das Dentalobjekt (100) und/oder ein Gehäuse (115) ein oder mehrere Kanäle (125) zum Führen von Speichel zu einer Sensoreinheit umfasst.
